# EUROPÄISCHE PATENTSCHRIFT

(11) **EP 3 009 088 B1**
(45) Veröffentlichungstag und Bekanntmachung des Hinweises auf die Patenterteilung: **08.05.2019**
(21) Anmeldenummer: 15189399.7
(22) Anmeldetag: 12.10.2015
(51) Int. Cl.: A61B 17/34

(54) **TROKARHÜLSE MIT EINER ASYMMETRISCHEN HELIX**
TROCAR SLEEVE WITH AN ASYMMETRIC HELIX
GAINE DE TROCART DOTEE D'UNE HELICE ASYMETRIQUE

(30) Priorität: 14.10.2014 DE 102014114890
(43) Veröffentlichungstag der Anmeldung: 20.04.2016
(73) Patentinhaber: Karl Storz SE & Co. KG, 78532 Tuttlingen (DE)
(72) Erfinder: Wagner, Sebastian, 75015 Bretten (DE); Oberländer, Martin, 78234 Engen (DE); Fuchs, Alexander, 78256 Steißlingen (DE)
(74) Vertreter: Witte, Weller & Partner Patentanwälte mbB

(56) Entgegenhaltungen:
- EP-A1- 2 747 678
- EP-A2- 2 529 684
- DE-A1- 10 156 312
- DE-U1- 9 102 553
- US-A- 5 630 813
- US-A- 5 895 351
- US-A1- 2003 153 926
- US-A1- 2012 209 205
- Heather Smith-Thomas: "Device helps cattle with bloat", Western Farmer-Stockman, 30 June 2010 (2010-06-30), page 10, XP055511091, Retrieved from the Internet: URL:http://magissues.farmprogress.com/WFS/ WS06Jun10/wfs010.pdf [retrieved on 2018-09-28]

## Beschreibung

Die Erfindung betrifft eine Trokarhülse, mit einem hohlen Schaft, der ein distales Ende und ein proximales Ende sowie eine etwa geradlinig verlaufende Schaftachse aufweist, wobei sich von der Außenseite des Schaftes ein Außengewinde erhebt, wobei am Schaft, von distal nach proximal gesehen, ein erstes Außengewinde in Form einer Helix vorhanden ist, deren Höhe H, um die sich diese von der Außenseite des Schaftes erhebt, zunimmt, und wobei, von distal nach proximal gesehen, die Steigung der Helix abnimmt.

Eine ähnliche Trokarhülse ist aus der EP 0 484 725 A bekannt. Weitere Gestaltungen von Trokarhülsen oder Kanülen sind beispielhaft aus der US 5,630,813 A, der DE 91 02 553 U1, sowie der DE 101 56 312 A1 bekannt.

Die US 5,630,813 A offenbart eine Kanüle mit einem Rohr, wobei ferner ein Hautverschluss in Form eines Kegels mit einem Außengewinde vorgesehen ist, der auf das Rohr der Kanüle aufschiebbar ist, wobei das Rohr relativ zum Hautverschluss axial beweglich ist.

Die DE 91 02 553 U1 offenbar ein Sicherungselement zur Begrenzung der Axialbewegung einer Trokarhülse mit einer Einspannung für die Trokarhülse und einer radialen Anlagefläche zur Abstützung des Sicherungselements gegen die Bauchdecke eines Patienten, wobei am distalen Ende des Sicherungselements eine zur Anlagefläche axial beabstandete Hintergreifung für die Bauchdecke ausgebildet ist, wobei die Hintergreifung ein Gewinde aufweist.

Die US 2003/153926 A1 offenbart eine Arthroskopiekanüle, mit einem länglichen Hohlschaft mit einem proximalen Ende und einem distalen Ende, einem Gewindebereich, der sich am distalen Ende des länglichen Hohlschaftes befindet, und mit einem verschiebbaren Halteelement, das auf dem Hohlschaft montiert ist.

Die DE 101 56 312 A1 offenbart einen Schaft für einen chirurgisch hergestellten Zugang, bei dem der Schaft an seinem distalen Ende an seiner Außenfläche einen Gewindesteg aufweist, welcher sich in Umfangsrichtung des Schaftes nur über einen Teilbereich des Umfanges erstreckt.

Trokarhülsen dienen dazu, bei einem minimalinvasiven Eingriff einen Zugang zu einer inneren Körperhöhle zu schaffen. Bei der weit verbreiteten Laparoskopie ist das die Bauchhöhle. Dazu weist die Trokarhülse einen hohlen Schaft auf, der, beim Setzen der Trokarhülse, über eine Inzision in der Bauchdecke angesetzt und durch diese hindurchgeschoben wird. Danach ragt das distale Ende des Schaftes in die Bauchhöhle hinein, das proximale Ende steht von der Oberseite der Bauchdecke ab.

Zum Setzen der Trokarhülse wird bei manchen Ausführungsformen ein Trokardorn in den hohlen Schaft eingeschoben, der distalseitig eine Spitze aufweist, die über das distale Ende der Trokarhülse hinaus reicht. Dieser Zusammenbau aus Trokarhülse und Trokardorn, also der eigentliche Trokar, wird durch die Bauchdecke hindurchgeschoben. Danach wird der Trokardorn abgezogen, so dass dann durch den hohlen Schaft entsprechende Instrumente in die Körperhöhle eingeführt werden können.

Bei manchen Trokarhülsen ist an der Außenseite des Hohlschaftes ein schraubenlinienförmig verlaufendes, sich von der Außenseite erhebendes Außengewinde vorhanden. Über dieses Gewinde kann die Trokarhülse durch das Gewebe hindurch eingedreht oder ausgedreht werden.

Insbesondere bei laparoskopischen Vorgängen ist es wünschenswert, die innere Körper-höhle, also die Bauchhöhle, mit einem Insufflationsgas aufzublähen, um eine bessere Sicht im Innenraum zu haben. Dazu ist meist am proximalen Ende des Schaftes der Trokarhülse ein Gehäuse angebracht, das mit einer Dichtung und mit einem Anschluss zum Zuführen eines Insufflationsgases versehen ist. Bei manchen Ausführungen können am Trokargehäuse noch weitere Anschlüsse vorhanden sein, beispielsweise um Spülflüssigkeiten zu- und abzuführen. Im praktischen Einsatz sind dann entsprechende Schlauchleitungen angeschlossen, wodurch die Trokarhülse relativ kopflastig wird. Dies beinhaltet die Gefahr, dass die Trokarhülse im Einsatz ihre Lage verändert, also beispielsweise sich nach proximal verschiebt oder verkippt. Ein versehentliches Herausgleiten der Trokarhülse aus dem Körper während eines endoskopischen Eingriffes wäre fatal.

Bei manchen Ausführungsformen besteht der hohle Schaft aus einem biegsamen Material, meist einem Kunststoffmaterial. Der lichte Innendurchmesser des hohlen Schaftes gibt den maximalen Durchmesser vor, den ein Instrument haben kann, das durch die Trokarhülse geführt werden kann. Bei manchen Operationstechniken ist es notwendig, gekrümmte oder seitlich ausgebogene Instrumente durchzuführen. Bei starren Trokarhülsen, beispielsweise aus Stahl, können solche Instrumente nicht oder nur unter Verformung hindurchgeführt werden. In diesen Fällen werden biegsame bzw. flexible Trokarhülsen eingesetzt. Der rohrförmige Körper des Hohlschaftes besteht aus einem elastischen Kunststoff, der sich der Biegung der durchgeführten Instrumente anpassen kann. Beim Durchführen von solchen gebogenen Instrumenten durch die bereits gesetzte Trokarhülse besteht die Gefahr, dass diese verschoben wird.

Außerdem weisen die eingesetzten Kunststoffmaterialien üblicherweise geringere Reibwerte als die Werkstoffe bei starren Trokarhülsen auf, so dass zusätzlich die Gefahr besteht, dass sich eine gesetzte Trokarhülse verschiebt.

Aus der EP 0 432 363 A sowie der EP 2 529 684 A sind Trokarhülsen bekannt, bei denen im distalen Endbereich der Trokarhülse, der in die Bauchhöhle ragt, Teile seitlich ausgespreizt werden können. Zusätzlich ist im proximalen Bereich ein Gegenhalter vorgesehen. Nach Setzen der Trokarhülse und Anlegen der gespreizten Bauelemente an die untere Innenseite der Bauchdecke kann von außen der Gegenhalter an die Bauchdecke angelegt und somit eine Fixierung der Trokarhülse bewerkstelligt werden.

Allerdings sind das aufwändig konstruierte Trokarhülsen, die manchmal schon im distalen Endbereich seitlich vorstehende Bauelemente haben, die schwierig über die Inzision durch die Bauchdecke, insbesondere nicht möglichst atraumatisch für den Patienten, einzubringen sind.

Es ist ferner ein laparoskopischer Veterinär-Trokar für Rinder der Firma Jorgensen Laboratories unter der Bezeichnung J 00 40 C bekannt, von dessen Außenseite sich ein Gewinde erhebt, dessen Höhe, von distal nach proximal gesehen, zunimmt.

Es ist Aufgabe der vorliegenden Erfindung, eine Trokarhülse dahingehend weiterzuentwickeln, die möglichst atraumatisch in eine Körperhöhle einbringbar ist und zumindest gegen Abziehen aus dem Körper gesichert ist.

Erfindungsgemäß wird die Aufgabe dadurch gelöst, dass der Schaft aus einem biegsamen Material in Form eines Kunststoffmaterials besteht, und dass, von distal nach proximal gesehen, ein Winkel α der proximalseitigen Flanke der Helix bezüglich der Schaftachse abnimmt und sich 90° annähert.

Dadurch, dass die Höhe der Helix, von distal nach proximal gesehen, zunimmt, kann die Trokarhülse durch Drehen und dabei gleichzeitigem Eindringen der Helix in das Gewebe besonders sanft eingeführt werden. Durch die nach und nach ansteigende Höhe der Helix kann das Gewebe im Bereich der Inzision sanft nach und nach immer weiter aufgeweitet werden. Durch die gleichzeitige Abnahme der Steigung der Helix kann zunächst zu Beginn des Eindrehens pro Drehung ein relativ großer axialer Vorschub erzielt werden. Da in diesem Bereich die Höhe der Helix noch relativ gering ist, ist dies besonders atraumatisch durchzuführen. Durch Abnahme der Steigung wird zwar beim fortschreitenden Eindrehen der Vorschub pro Drehung geringer, dies fördert aber ein sanftes Aufweiten und Durchdrehen der Helix durch die Bauchdecke hindurch.

Ist die Helix komplett durch die Bauchdecke durchgedreht, steht der Unterseite der Bauchdecke derjenige Abschnitt der Helix gegenüber, der die größte Höhe aufweist. Dadurch wird einem Abziehen der Trokarhülse durch Zugbewegungen, wie sie bei der Manipulation am Trokargehäuse bei einem medizinischen Eingriff auftreten, effektiv entgegengewirkt. Kräfte, die eine Drehung der Trokarhülse bewirken, treten bei solchen Manipulationen oder medizinischen Eingriffen normalerweise nicht auf.

Somit kann die Trokarhülse im Bereich der Helix, obwohl diese eine zunehmende Höhe aufweist, ohne große Beeinträchtigungsgefahr für den Patienten eingedreht werden und entfaltet, nach Durchdrehen der Helix durch die Bauchdecke, eine erhebliche Widerstandskraft gegen axial gerichtete Abziehbewegungen.

Erfindungsgemäß nimmt der Winkel der proximalseitigen Flanke der Helix, von distal nach proximal gesehen, bezüglich der Schaftachse ab und nähert sich etwa 90°.

Diese Maßnahme hat den Vorteil, dass nach Durchdrehen der Helix der Unterseite der Bauchdecke eine Flanke entgegensteht, die sich etwa parallel zur Richtung der Unterseite der Bauchdecke erstreckt, so dass eine besonders gute Sperrwirkung gegenüber axialem Abziehen in proximaler Richtung vorhanden ist. Dennoch ist der Flankenwinkel im Zusammenhang mit der Steigung noch so ausgeprägt vorhanden, dass die Trokarhülse nach Beendigen des medizinischen Eingriffes wieder aus der Bauchdecke ausgedreht werden kann. In Kombination mit der maximalen Höhe der Helix steht somit eine günstig ausgerichtete Sperrfläche zur Verfügung, die mit der Innenseite des Gewebes der Körper-höhle sperrend ein Eingriff treten kann.

in einer weiteren Ausgestaltung der Erfindung steigt die Höhe der Helix von Null bis zu einer maximalen Höhe an.

Diese Maßnahme hat den Vorteil, dass die Trokarhülse zunächst an die entsprechende Inzision angesetzt und so weit linear vorgeschoben werden kann, bis die Helix beginnt anzusteigen. Ab diesem Zeitpunkt kann dann die Trokarhülse im Bereich der Helix einfach und relativ atraumatisch durch das Gewebe hindurchgedreht werden. Der Anstieg von der Höhe Null schafft ein besonders sanftes Hineindrehen der Helix in die Gewebeöffnung. Die maximale Höhe der Helix richtet sich nach der Größe der Trokarhülse und auch der Art des Eingriffes, insbesondere welche Anschlüsse und Manipulationen am proximalen Ende des Trokares erfolgen, die die Gefahr eines proximalen Abziehens beinhalten.

In einer weiteren Ausgestaltung der Erfindung ist der Anstieg der Höhe der Helix stetig.

Diese Maßnahme hat den Vorteil, dass der Eindrehvorgang besonders sanft, insbesondere ruckfrei, durchgeführt werden kann.

In einer weiteren Ausgestaltung der Erfindung beträgt die maximale Höhe der Helix bis zum Maß des Außendurchmessers des Trokarschaftes.

In diesem Höhenbereich kann eine besonders sichere Fixierung der Trokarhülse gegen versehentliches Abziehen erzielt werden.

In einer weiteren Ausgestaltung der Erfindung nimmt die Höhe der Helix nach Überschreiten der maximalen Höhe spätestens innerhalb einer 360°-Windung wieder auf Null ab.

Diese Maßnahme hat den Vorteil, dass nach Durchdrehen des Bereiches mit maximaler Höhe die Trokarhülse noch etwas weitergedreht werden kann, so dass sich dann alsbald das Gewebe hinter der eingedrehten Helix sanft an die Außenseite des Schaftes anlegen kann. In anderen Worten ausgedrückt ist am Ende der Helix keine Stufe vorhanden. Diese Maßnahme erleichtert auch das Ausdrehen der Trokarhülse nach dem medizinischen Eingriff.

In einer Ausgestaltung verändert sich der Winkel der Steigung der Helix aus einer Ebene senkrecht zur Schaftachse, von distal nach proximal gesehen, von einem spitzen Winkel größer 45° bis auf einen Winkel kleiner 20°.

Daraus resultiert eine Scheitelkurve der Helix, in der alle Scheitelpunkte des Gewindes liegen, die das atraumatische Eindrehen des Gewindes durch das Gewebe fördert.

In einer weiteren Ausgestaltung der Erfindung ist distalseitig der Helix ein Schaftabschnitt ohne Außengewinde vorhanden.

Diese Maßnahme hat den Vorteil, dass dieser "glatte" Abschnitt ohne Außengewinde zunächst "klassisch" angesetzt und in einem axialen Verschiebevorgang nach distal durch das Gewebe eingetrieben werden kann. Zu diesem Zeitpunkt kann auch schon ein Trokardorn abgezogen werden. Hat das distalseitige Ende der Helix die Außenseite des Gewebes erreicht, kann der Drehvorgang begonnen und die Trokarhülse im Bereich der Helix durch das Gewebe, beispielsweise die Bauchdecke, gedreht werden. Dabei ist von besonderem Vorteil, dass sich die Helix nur über einen Abschnitt des Schaftes erstreckt.

In einer weiteren Ausgestaltung der Erfindung ist proximalseitig der Helix am Schaft ein längs der Schaftachse bewegbarer Gegenhalter angeordnet, der an eine Oberseite eines Gewebes, durch das die Trokarhülse durchgeschoben ist, anlegbar ist.

Durch diese an sich bekannte Maßnahme kann eine verbesserte Fixierung der Trokarhülse erzielt werden, insbesondere ein versehentliches weiteres Einschieben der Trokarhülse nach distal zusätzlich verhindert werden.

Diese Sicherung könnte prinzipiell auch dadurch erreicht werden, dass die Helix nicht vollständig durch den Gewebebereich, sprich die Bauchdecke, hindurchgedreht wird, sondern Gewindeabschnitte der Helix mit relativ großer Höhe im Bereich der Öffnung verbleiben, die sowohl an der Unterseite als auch an der Oberseite anliegen. Das würde allerdings das Gewebe im Bereich der Öffnung, durch die die Trokarhülse hindurch-gebracht wird, zusätzlich strapazieren.

Es ist daher vorteilhaft, die gesamte Helix durch die Bauchdecke hindurchzudrehen, so dass über die Höhe der Bauchdecke gesehen nur ein glatter gewindefreier Abschnitt des Schaftes im Bereich der Gewebeöffnung verbleibt. Daher wird proximalseitig der Helix ein solcher gewindefreier Abschnitt vorgesehen.

In einer weiteren Ausgestaltung der Erfindung ist der Gegenhalter als scheibenartiges Element ausgebildet, das längs der Schaftachse verschiebbar und in zumindest einer Stellung ortsfest am Schaft haltbar ist.

Diese Maßnahme hat den Vorteil, dass das Anlegen und Arretieren des Halters besonders einfach und vor allem von der Außenseite, also für die Handhabungsperson gut ersichtlich durchführbar ist.

In einer weiteren Ausgestaltung der Erfindung weist der Gegenhalter ein Innengewinde auf, das mit einem entsprechenden zweiten Außengewinde an der Außenseite des Schaftes kämmt, so dass proximalseitig der Helix der Gegenhalter in axialer Richtung hin und her bewegbar ist.

In einer weiteren Ausgestaltung der Erfindung ist der Gegenhalter aus einem weichelastischen Kunststoffmaterial hergestellt und der Gegenhalter ist über ein zweites Außengewinde an der Außenseite des Schaftes axial hin- und herbewegbar.

Diese Maßnahme hat den Vorteil, dass aufgrund der weichelastischen Materialbeschaffenheit der Gegenhalter einfach über seine Öffnung auf den Schaft aufgeschoben und im Bereich des zweiten Außengewindes bei den axialen Verschiebebewegungen gezielt geführt werden kann. Fertigungstechnisch kann das Innengewinde in der Öffnung des Gegenhalters weggelassen werden. Ein so ausgestalteter Gegenhalter kann auch auf einen Schaft ohne zweites Außengewinde aufgeschoben und längs diesem bewegt werden. Die Öffnung im Gegenhalter ist dann so bemessen, dass diese durch den Schaft etwas aufgeweitet wird und der Gegenhalter reibschlüssig auf dem Schaft sitzt und unter Überwindung der Reibkraft verschoben werden kann.

Der Gegenhalter kann auch als geschlitztes oder U-förmiges Element ausgebildet sein, das seitlich auf den Schaft angeclipst werden kann.

Es ist von Vorteil, im Bereich zwischen der Helix und dem zweiten Außengewinde einen gewindefreien Abschnitt vorzusehen. In diesem glatten Außenflächenbereich des Schaftes kann sich das Gewebe sanft anlegen. Bei der geschlitzten Ausführung des Gegenhalters kann dieser in diesen Bereich seitlich aufgeclipst werden.

Es versteht sich, dass die vorstehend und die nachstehend noch genannten Merkmale nicht nur in den angegebenen Kombinationen, sondern auch in anderen Kombinationen einsetzbar sind, ohne den Rahmen der vorliegenden Erfindung zu verlassen.

Die Erfindung wird nachfolgend in Zusammenhang mit den beiliegenden Zeichnungen näher beschrieben und erläutert. Es zeigen:
- Fig. 1: perspektivisch eine erfindungsgemäße Trokarhülse ohne proximale Dichtung,
- Fig. 2: eine Seitenansicht der Trokarhülse mit einer proximalen Dichtung,
- Fig. 2a: eine ausschnittsweise Seitenansicht zur Erläuterung des Winkels der Steigung der Helix
- Fig. 3: einen Längsschnitt längs der Linie III-III in Fig. 2 im Bereich der Helix,
- Fig. 4: einen Querschnitt längs der Linie IV-IV in Fig. 2,
- Fig. 5: einen Querschnitt längs der Linie V-V in Fig. 2,
- Fig. 6: einen Querschnitt längs der Linie VI-VI in Fig. 2,
- Fig. 7: einen Querschnitt längs der Linie VII-VII in Fig. 2,
- Fig. 8: einen Querschnitt längs der Linie VIII-VIII in Fig. 2,
- Fig. 9: eine teilweise Seitenansicht der Trokarhülse mit einem eingeschobenen Trokar-dorn kurz vor dem Setzen an einer Bauchdecke,
- Fig. 10: eine der Fig. 9 entsprechende Darstellung in einer Situation, bei der ein distaler Endabschnitt des Schaftes schon durch die Bauchdecke hindurchgeschoben ist und der Trokar abgezogen ist,
- Fig. 11: eine der Fig. 10 vergleichbare Situation zu Beginn des Eindrehens der Helix in die Bauchdecke,
- Fig. 12: eine Situation, bei der die Helix vollständig durch die Bauchdecke hindurch-gedreht ist, und
- Fig. 13: eine Situation, bei der ein Gegenhalter an die Außenseite der Bauchdecke angelegt ist.

Eine in den Figuren dargestellte Trokarhülse ist in ihrer Gesamtheit mit der Bezugsziffer 10 bezeichnet.

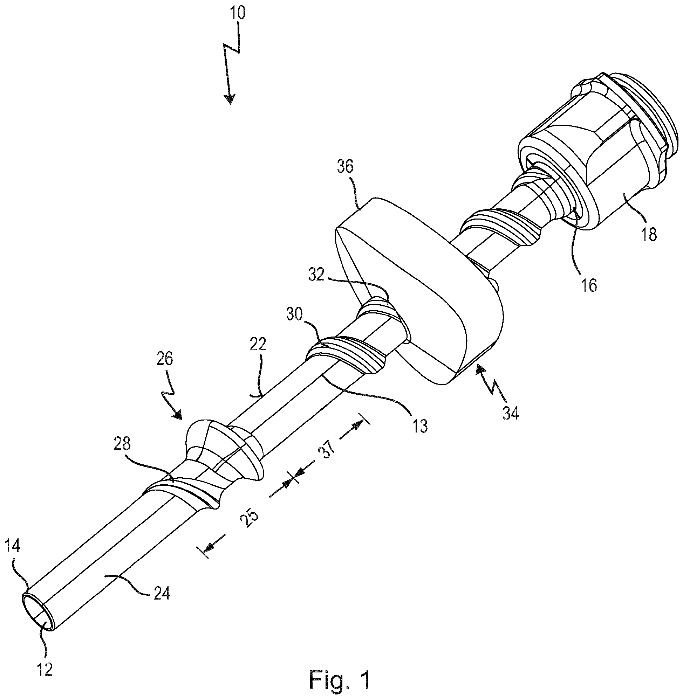

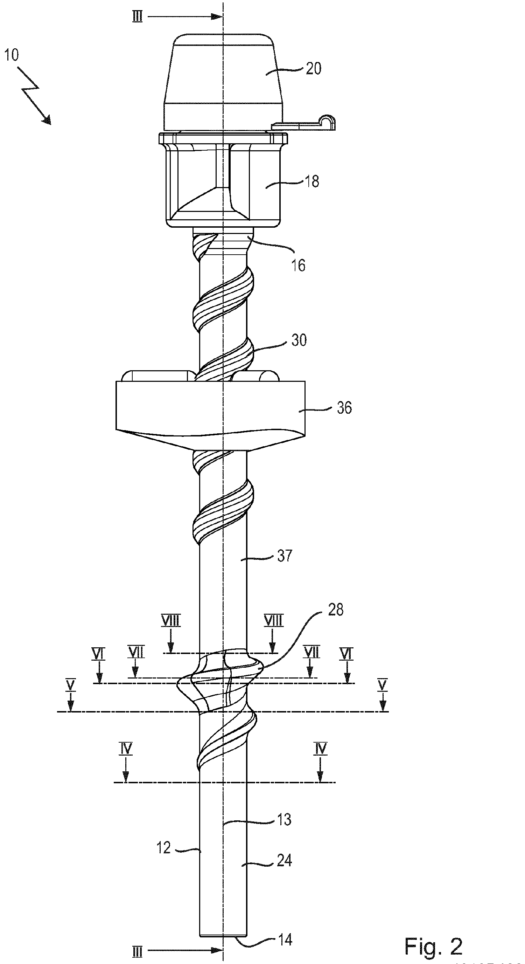

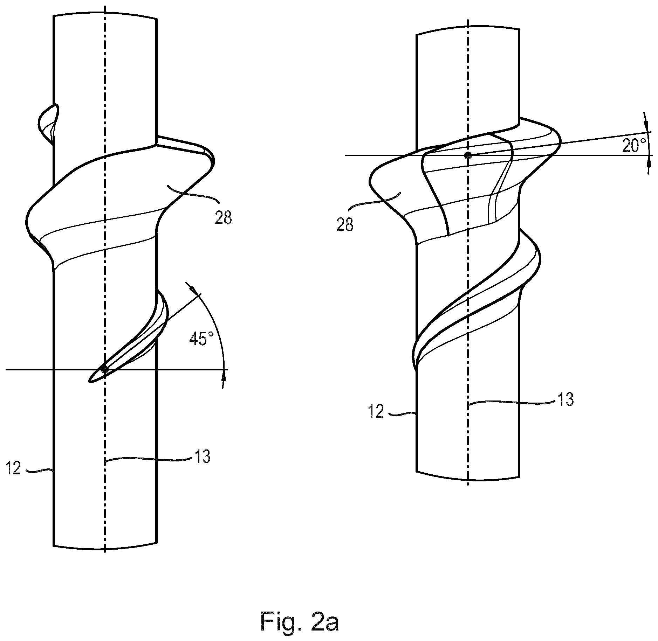

Wie insbesondere aus den Fig. 1 und 2 zu erkennen, weist die Trokarhülse 10 einen sich geradlinig erstreckenden Hohlschaft 12 auf, der eine Schaftachse 13 aufweist. Der Schaft 12 weist eine zylindrische Außenseite 22 auf.

Der Schaft 12 weist ein offenes distales Ende 14 auf und ist an seinem proximalen Ende mit einem über eine Dichtung 20 (Fig. 2) gasdicht abschließbaren Gehäuse 18 versehen. Aus den Fig. 1 und 2 ist zu erkennen, dass die Außenseite 22 in einem distalen Endabschnitt 24 des Schaftes 12 glatt ist, also kein Gewinde aufweist.

An diesen distalen Endabschnitt 24 schließt sich proximalseitig ein Längenabschnitt 25 an, an dem ein erstes Außengewinde 26 in Form einer Helix 28 vorhanden ist, wobei sich die Helix 28 von der zylindrischen Außenseite 22 des Schaftes 12 erhebt.

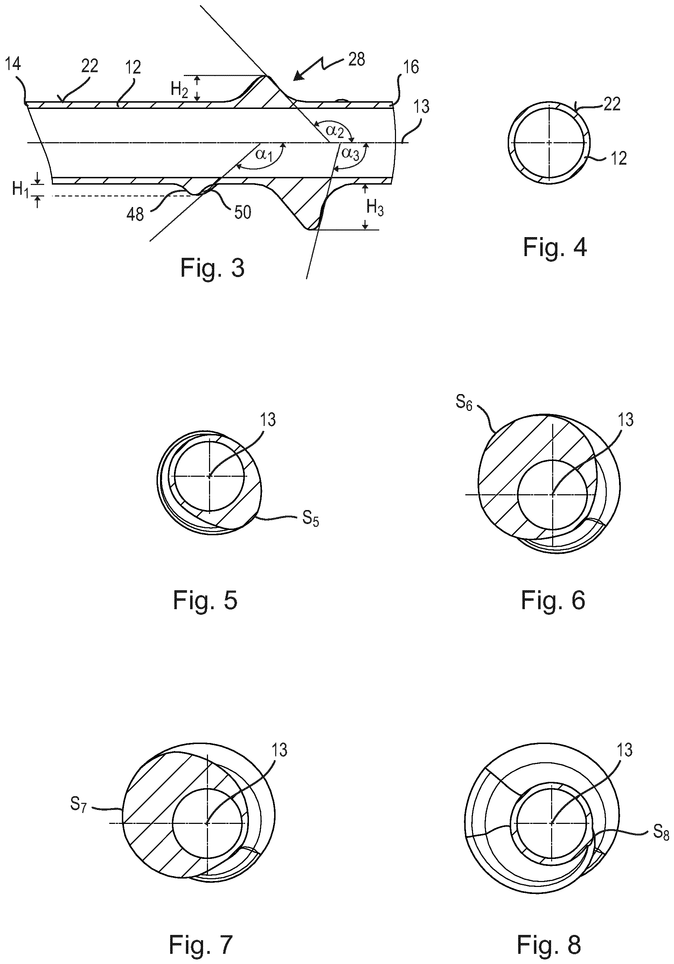

Die nähere Ausgestaltung der Helix 28 soll zunächst insbesondere in Zusammenhang mit Fig. 2 und den Schnittdarstellungen von Fig. 3 bis 8 näher beschrieben und erläutert werden.

Die Helix 28 ist im gezeigten Ausführungsbeispiel aus einer schraubenlinienförmigen Windung aufgebaut, die etwa zwei vollständige Gewindegänge aufweist. Die Helix bzw. die Scheitelkurve des ersten Außengewindes 26, also die Kurve, die alle Scheitelpunkte des Gewindes enthält, weist einen Steigungswinkel aus einer Ebene senkrecht zur Schaftachse, von distal nach proximal gesehen, auf, der sich von einem spitzen Winkel größer 45° bis auf einen Winkel kleiner 20° verändert.

Aus den Schnittdarstellungen von Fig. 3 und 4 ist zu erkennen, dass, vom distalen Ende 14 Richtung des proximalen Endes 16 gesehen, die Helix 28 aus einer Höhe Null über eine Höhe H₁ über eine Höhe H₂ bis zu einer maximalen Höhe H₃ ansteigt. Der Anstieg ist dabei stetig.

Die Helix beginnt bei einer Höhe Null, steigt dann alsbald auf eine Höhe von etwa 2,7 mm an, hat im Bereich des Schnittes von Fig. 5 eine Höhe von 5 mm und in Höhe der Schnitte der Fig. 6 und 7 eine Höhe von 6,5 mm. Nach Überschreiten der maximalen Höhe H₃ von etwa 6,5 mm nimmt die Höhe sehr rasch (ca. nach 90°) wieder ab, und zwar auf eine sehr geringe Höhe von etwa 2,7 mm, wie das in Fig. 8 dargestellt ist.

Die jeweiligen Höhen sind in den Fig. 5 bis 8 durch die Scheitelpunkte S5, S6, S7 und S8 angedeutet.

Nimmt man nunmehr die Fig. 2 heran, so ist zu erkennen, dass die Steigung der Helix, von distal nach proximal, laufend abnimmt. Die Steigung ist definiert durch den Höhenunterschied zweier gegenüberliegender Scheitelpunkte nach einer 360°-Drehung.

Die Steigung zu Beginn der Helix 28, also nach dem Schnitt von Fig. 4, liegt bei 20 mm pro Umdrehung, die Steigung im Bereich des Schnittes von Fig. 5 im Bereich von 10 mm pro Umdrehung, die Steigung im Bereich des Schnittes von Fig. 6 im Bereich von 7 mm pro Umdrehung und im Bereich des Schnittes von Fig. 7 nur noch 4 mm pro Umdrehung. Nach Überschreiten des Maximums beträgt die Steigung 8 mm pro Umdrehung.

Der Begriff "pro Umdrehung" ist so zu verstehen, dass, wenn man diesen Punkt nimmt und eine vollständige Umdrehung machen würde, diese Steigung resultieren würde.

Faktisch ändert sich die Steigung der Helix 28 jedoch laufend von distal nach proximal gesehen.

Insbesondere aus der Schnittdarstellung von Fig. 3 ist zu erkennen, dass der Winkel der distalseitigen Flanke 48 der Helix 28 nahezu unverändert bleibt.

Der Winkel der Flanke 50, die dem proximalen Ende zugewandt ist, nimmt aber laufend ab. Der Flankenwinkel α der Flanke 50 ist der Winkel zwischen der Flanke 50 und, von distal nach proximal gesehen, der Schaftachse 13. So beträgt der Flankenwinkel α₁ zu Beginn der Helix bezüglich der Schaftachse 13, von distal nach proximal gesehen, etwa 140°. Dieser Flankenwinkel nimmt ab, so dass beispielsweise der Flankenwinkel α₂ etwa bei 130° liegt. Im Bereich der letzten Windung, also im Bereich der höchsten Höhe H₃, beträgt der Flankenwinkel α₃ knapp 100°.

Insgesamt gesehen steigt somit die Höhe H der Helix 28 in dem Schaftabschnitt 25 von Null auf die maximale Höhe H₃ an und fällt danach rasch ab. Der gesamte Gewindegang der Helix 28 beinhaltet etwa zwei vollständige 360°-Umdrehungen.

Gleichzeitig nimmt die Steigung, von distal nach proximal gesehen, ab, und der Winkel der Flanke der Helix in Bezug auf die Schaftachse 13, die dem proximalen Ende zugewandt ist, wird immer geringer.

Zunächst zurückkehrend zu den Fig. 1 und 2 ist zu erkennen, dass sich an den Schaftabschnitt 25 mit der Helix 28 ein gewindefreier Abschnitt 37 erstreckt. Anschließend an diesen gewindefreien Abschnitt 37 erhebt sich von der Außenseite 22 des Schaftes ein zweites Außengewinde 30, das aber als ein "regelmäßiges" Außengewinde mit gleichbleibender Höhe, gleichbleibender Steigung und gleichbleibender Flankenausgestaltung ausgebildet ist.

Auf diesem zweiten Außengewinde 30 ist ein Gegenhalter 34 in Form einer dreieckigen Scheibe 36 aufgenommen. Der Gegenhalter 34 ist aus einem weichelastischen Silikonmaterial hergestellt. Die dreieckige Scheibe 36 kann in axialer Richtung nach proximal oder distal bewegt werden. Die Dreiecksform begünstigt das Ergreifen und Drehen bzw. Verschieben des Gegenhalters. Das weichelastische Silikonmaterial legt sich im Bereich dessen mittiger Öffnung 32 unter Verformung an die Kontur des zweiten Außengewindes 30 an.

In der Figurenfolge 9 bis 13 soll eine beispielhafte Anwendung der erfindungsgemäßen Trokarhülse 10 bei der Laparoskopie beschrieben werden.

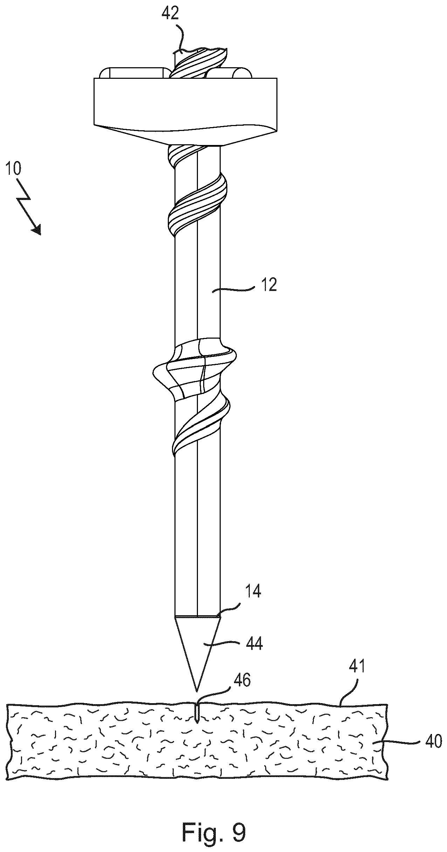

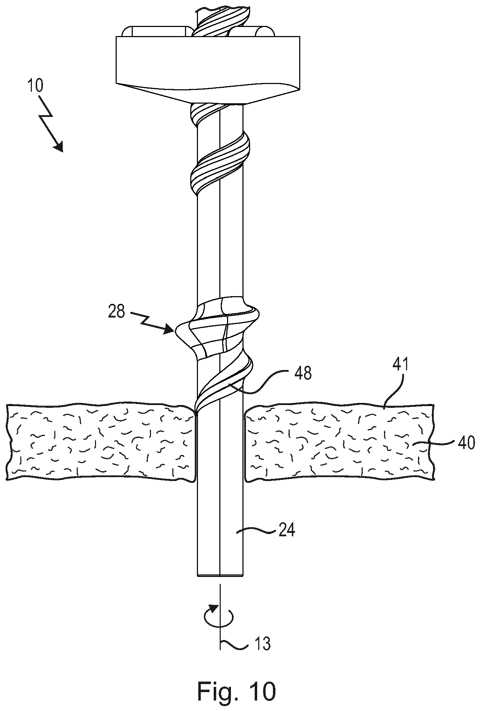

Wie aus Fig. 9 ersichtlich, ist dazu in dem inneren Hohlraum des Schaftes 12 ein Trokardorn 42 eingeschoben, dessen Spitze 44 über das distale Ende der Trokarhülse 10 hinausreicht. Diese Spitze 44 wird an einer Inzision 46 an der Ober- bzw. Außenseite 41 einer Bauchdecke 40 angesetzt. Durch axiales Vorschieben dieses Zusammenbaus wird die Trokarhülse 10 zunächst über den glatten distalen Endabschnitt 24 durch die Bauchdecke 40 hindurchgeschoben, bis die Helix 28 die Oberseite 41 erreicht, wie das in Fig. 10 dargestellt ist. Der Trokardorn 42 kann nunmehr abgezogen werden.

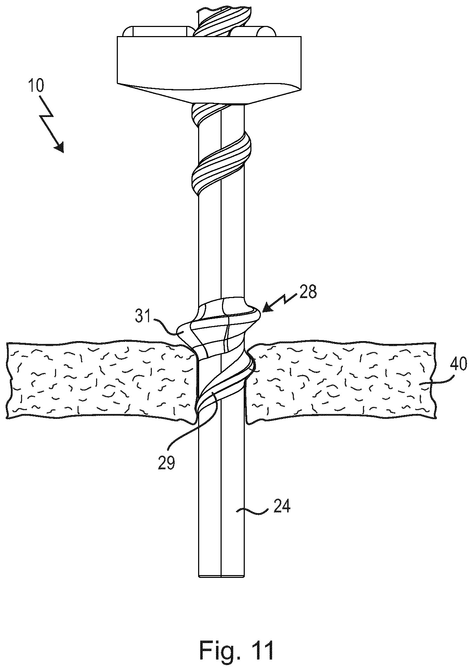

Nunmehr wird, wie das aus dem Übergang von Fig. 10 zur Fig. 11 ersichtlich ist, die Helix 28 nach und nach durch die Bauchdecke 40 hindurchgedreht. Es tritt also zunächst der Bereich der Helix 28 in die Bauchdecke ein, der mit der geringen Höhe, aber der hohen Steigung versehen ist. Dadurch kann die Trokarhülse 10 durch eine 360°-Umdrehung um einen gewissen Abschnitt eingedreht werden, wie das aus dem Übergang von Fig. 10 zu Fig. 11 ersichtlich ist.

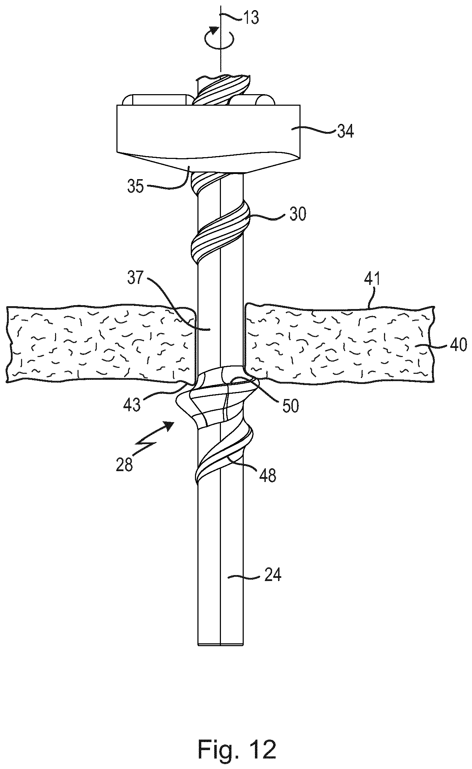

Durch weiteres Drehen der Trokarhülse 10 um die Schaftachse 13 wird die Helix 28 weiter eingedreht und komplett durch die Bauchdecke 40 durchgedreht. Dies ist in Fig. 12 dargestellt. Es ist ersichtlich, dass dazu zumindest eine weitere 360°-Umdrehung notwendig war. Das erste Außengewinde 26 weist also nur etwa zwei Windungen auf, deren gesamte Ganghöhe in etwa der Dicke eines Gewebes entspricht, durch das die Trokarhülse durchgedreht werden soll.

Aus Fig. 11 ist ersichtlich, dass in Richtung der Längsachse des Schaftes gesehen, relative große Bereiche der zylindrischen Außenseite des Schaftes zwischen den Windungen 29 und 31 vorhanden sind, an die sich das Gewebe der Bauchdecke auch während dem Eindrehen anlegen kann.

Es muss weniger Gewebe dilatiert werden, somit entsteht auch weniger Spannung und das Durchdrehen der Helix erfordert einen geringeren Kraftaufwand. Dadurch sinkt auch die Torsionsbelastung im Schaft. Das eröffnet die Möglichkeit, den Schaft aus biegsamem Kunststoffmaterial herzustellen.

Das eröffnet auch die Möglichkeit, die Trokarhülse in einem Blasformverfahren herzustellen, bei dem zugleich auch das/die Außengewinde ausgeformt werden.

Nunmehr steht die proximalseitige Flanke 50 mit dem geringen Neigungswinkel α₃ (siehe Fig. 3) der Unterseite 43 der Bauchdecke 40 gegenüber. Zugleich ist die Höhe H der Helix 28 in diesem Bereich am ausgeprägtesten. Somit ist aus Fig. 12 deutlich ersichtlich, dass einem axialen Verschieben der Trokarhülse 10 nach proximal die Helix 28 erhebliche Widerstände entgegensetzt.

Dadurch ist ausgeschlossen, dass bei den üblichen Manipulationen die Trokarhülse 10 versehentlich von der Bauchdecke 40 abgezogen wird.

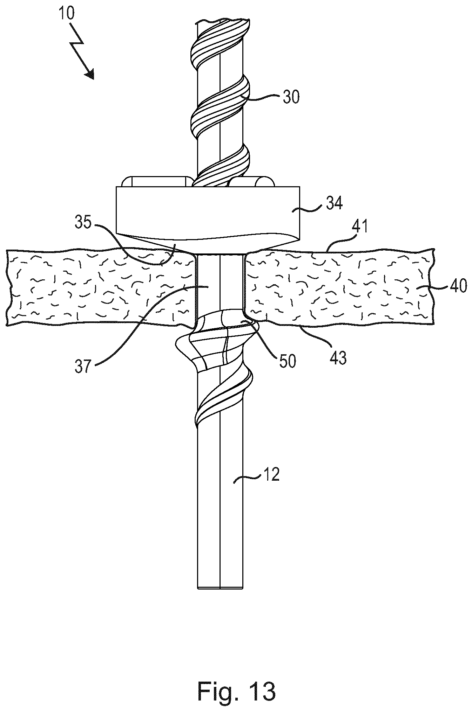

Als zusätzliche Sicherheit zur Fixierung ist der Gegenhalter 34 vorgesehen, der dann so weit um das zweite Außengewinde 30 gedreht werden kann, bis dessen Unterseite auf der Oberseite 41 der Bauchdecke 40 zum Liegen kommt, wie das in Fig. 13 dargestellt ist.

In dieser Position ist die Trokarhülse 10 gegen axiales Verschieben sowohl nach proximal als auch nach distal gesichert und wird auch relativ kippstabil gehalten. Aus Fig. 13 ist zu erkennen, dass sich das Gewebe im Bereich der Öffnung in der Bauchdecke 40 um den gewindefreien glatten Abschnitt 37 zwischen dem Ende der Helix 28 und dem Beginn des zweiten Außengewindes 30 gelegt hat. Das heißt, auch bei lang anhaltenden Eingriffen erfolgt keine Beeinträchtigung des Gewebes durch die beiden Außengewinde 26 und 30.

Nach Beendigung des operativen Eingriffes wird die Trokarhülse 10 wieder aus der Bauchdecke 40 herausgedreht, wobei dies dadurch erleichtert wird, dass die Helix 28 proximalseitig der höchsten Erhebung bis auf die Höhe Null, allerdings mit geringer Steigung, noch etwas weitergeführt ist.

## Patentansprüche

1. Trokarhülse, mit einem Hohlschaft (12), der ein distales Ende (14) und ein proximales Ende (16) sowie eine etwa geradlinig verlaufende Schaftachse (13) aufweist, wobei sich von der Außenseite (22) des Schaftes (12) ein Außengewinde erhebt, wobei am Schaft (12), von distal nach proximal gesehen, ein erstes Außengewinde (26) in Form einer Helix (28) vorhanden ist, deren Höhe (H), um die sich diese von der Außenseite (22) des Schaftes (12) erhebt, zunimmt, und wobei, von distal nach proximal gesehen, die Steigung der Helix (28) abnimmt, wobei der Schaft (12) aus einem biegsamen Material in Form eines Kunststoffmaterials besteht, und wobei von distal nach proximal gesehen, ein Winkel (α) der proximalseitigen Flanke (50) der Helix (28) bezüglich der Schaftachse (13) abnimmt und sich 90° annähert.

2. Trokarhülse nach Anspruch 1, **dadurch gekennzeichnet, dass** die Höhe (H) der Helix (28) von Null bis zu einer maximalen Höhe (H₃) ansteigt.

3. Trokarhülse nach einem der Ansprüche 1 oder 2, **dadurch gekennzeichnet, dass** der Anstieg der Höhe (H) stetig ist.

4. Trokarhülse nach Anspruch 2 oder 3, **dadurch gekennzeichnet, dass** die maximale Höhe (H₃) der Helix (28) bis zum Maß des Außendurchmessers des Trokarschaftes (12) betragen kann.

5. Trokarhülse nach einem der Ansprüche 2 bis 4, **dadurch gekennzeichnet, dass** die Höhe (H) der Helix (28), nach Überschreiten der maximalen Höhe (H₃), spätestens innerhalb einer 360°-Windung wieder auf Null abnimmt.

6. Trokarhülse nach einem der Ansprüche 1 bis 5, **dadurch gekennzeichnet, dass** sich der Winkel der Steigung der Helix (28), aus einer Ebene senkrecht zur Schaftachse (13), von distal nach proximal gesehen, von einem spitzen Winkel größer 45° bis auf einen Winkel kleiner 20° verändert.

7. Trokarhülse nach einem der Ansprüche 1 bis 6, **dadurch gekennzeichnet, dass** distalseitig der Helix (28) ein Schaftabschnitt (24) ohne Außengewinde vorhanden ist.

8. Trokarhülse nach einem der Ansprüche 1 bis 7, **dadurch gekennzeichnet, dass** sich die Helix (28) nur in einem Abschnitt (25) des Schaftes (12) erstreckt.

9. Trokarhülse nach einem der Ansprüche 1 bis 8, **dadurch gekennzeichnet, dass** proximalseitig der Helix (28) am Schaft (12) ein längs zur Schaftachse (13) bewegbarer Gegenhalter (34) angeordnet ist, der an eine Oberseite eines Gewebes, durch das die Trokarhülse durchgeschoben ist, anlegbar ist.

10. Trokarhülse nach Anspruch 9, **dadurch gekennzeichnet, dass** der Gegenhalter (34) als scheibenartiges Element (36) ausgebildet ist, das längs der Schaftachse (13) verschiebbar und in zumindest einer Stellung ortsfest am Schaft (12) haltbar ist.

11. Trokarhülse nach Anspruch 9 oder 10, **dadurch gekennzeichnet, dass** der Gegenhalter (34) ein Innengewinde aufweist, das über ein entsprechendes zweites Außengewinde (30) an der Außenseite (22) des Schaftes proximalseitig der Helix (28) axial hin und her verschiebbar ist.

12. Trokarhülse nach Anspruch 9 oder 10, **dadurch gekennzeichnet, dass** der Gegenhalter (34) aus einem weichelastischen Kunststoffmaterial hergestellt ist, und dass der Gegenhalter (34) über ein zweites Außengewinde (30) an der Außenseite (22) des Schaftes (12) axial hin- und herverschiebbar ist.

13. Trokarhülse nach Anspruch 12, **dadurch gekennzeichnet, dass** der Gegenhalter als geschlitztes Element ausgebildet ist, das seitlich auf den Schaft (12) aufclipsbar ist.

14. Trokarhülse nach einem der Ansprüche 11 bis 13, **dadurch gekennzeichnet, dass** zwischen der Helix (28) und dem zweiten Außengewinde (30) ein gewindefreier Schaftabschnitt (37) vorhanden ist.

15. Trokarhülse nach einem der Ansprüche 1 bis 14, **dadurch gekennzeichnet, dass** der Schaft (12) eine zylindrische Außenseite (22) aufweist.

16. Trokarhülse nach einem der Ansprüche 1 bis 15, **dadurch gekennzeichnet, dass** das erste Außengewinde (26) etwa zwei Windungen aufweist, deren gesamte Ganghöhe in etwa der Dicke eines Gewebes entspricht, durch das die Trokarhülse durchgedreht werden soll.

## Claims

1. A trocar sleeve, comprising a hollow shaft (12) which has a distal end (14) and a proximal end (16) and an approximately rectilinear shaft axis (13), an external thread rising from the outer face (22) of the shaft (12), wherein at the shaft (12), seen from distal to proximal, a first external thread (26) in the form of a helix (28) is provided, the height (H) of which, by which it rises from the outer face (22) of the shaft (12), increases, and wherein, seen from distal to proximal, the pitch of the helix (28) decreases, wherein the shaft (12) consists of a flexible material in the form of a plastic material, and wherein, seen from distal to proximal, an angle (α) of the proximal flank (50) of the helix (28) with respect to the shaft axis (13) decreases and approaches 90°.

2. The trocar sleeve according to claim 1, **characterized in that** the height (H) of the helix (28) increases from zero to a maximum height (H₃).

3. The trocar sleeve according to any one of claims 1 or 2, **characterized in that** the increase in the height (H) is continuous.

4. The trocar sleeve according to claim 2 or 3, **characterized in that** the maximum height (H₃) of the helix (28) can amount up to the extent of the external diameter of the trocar shaft (12).

5. The trocar sleeve according to any one of claims 2 to 4, **characterized in that** the height (H) of the helix (28), having passed the maximum height (H₃), decreases to zero again within at most a 360° winding.

6. The trocar sleeve according to any one of claims 1 to 5, **characterized in that** the angle of the pitch of the helix (28), from a plane perpendicular to the shaft axis (13), seen from proximal to distal, changes from an acute angle of more than 45° to an angle of less than 20°.

7. The trocar sleeve according to any one of claims 1 to 6, **characterized in that** a shaft portion (24) without an external thread is present distal to the helix (28).

8. The trocar sleeve according to any one of claims 1 to 7, **characterized in that** the helix (28) extends only in a portion (25) of the shaft (12).

9. The trocar sleeve according to any one of claims 1 to 8, **characterized in that** a counter holder (34), which is movable along the shaft axis (13) and which can be placed on an upper face of a tissue through which the trocar sleeve is pushed, is arranged on the shaft (12) proximal to the helix (28).

10. The trocar sleeve according to claim 9, **characterized in that** the counter holder (34) is designed as a disc-like element (36) which is movable along the shaft axis (13) and which can be held stationary on the shaft (12) in at least one position.

11. The trocar sleeve according to claim 9 or 10, **characterized in that** the counter holder (34) has an internal thread which is movable axially to and fro over a corresponding second external thread (30) on the outer face (22) of the shaft on the proximal side of the helix (28).

12. The trocar sleeve according to claim 9 or 10, **characterized in that** the counter holder (34) is made from a soft elastic plastic material, and **in that** the counter holder (34) is movable axially to and fro over a second external thread (30) on the outer face (22) of the shaft (12).

13. The trocar sleeve according to claim 12, **characterized in that** the counter holder is designed as a slit element that can be clipped laterally onto the shaft (12).

14. The trocar sleeve according to any one of claims 11 to 13, **characterized in that** a thread-free shaft portion (37) is present between the helix (28) and the second external thread (30).

15. The trocar sleeve according to any one of claims 1 to 14, **characterized in that** the shaft (12) has a cylindrical outer face (22).

16. The trocar sleeve according to any one of claims 1 to 15, **characterized in that** the first external thread (26) has approximately two turns, the total pitch of which corresponds approximately to the thickness of a tissue through which the trocar sleeve is to be rotated.

## Revendications

1. Gaine de trocart, avec une tige creuse (12), qui présente une extrémité distale (14) et une extrémité proximale (16) ainsi qu'un axe de tige (13) s'étendant environ en ligne droite, dans laquelle un filet extérieur est saillant sur le côté extérieur (22) de la tige (12), dans laquelle il se trouve, en regardant de distal vers proximal, un premier filet extérieur (26) en forme d'hélice (28), dont la hauteur (H), dont celle-ci est saillante sur le côté extérieur (22) de la tige (12), augmente et dans laquelle, en regardant de distal vers proximal, le pas de l'hélice (28) diminue, dans laquelle la tige (12) est constituée d'un matériau flexible sous la forme d'un matériau à base de matière plastique, et dans laquelle, en regardant de distal vers proximal, un angle (a) du flanc côté proximal (50) de l'hélice (28) par rapport à l'axe de tige (13) diminue et s'approche de 90°.

2. Gaine de trocart selon la revendication 1, **caractérisée en ce que** la hauteur (H) de l'hélice (28) augmente de zéro à une hauteur maximale (H₃).

3. Gaine de trocart selon une des revendications 1 ou 2, **caractérisée en ce que** la hausse de la hauteur (H) est continue.

4. Gaine de trocart selon une revendication 2 ou 3, **caractérisée en ce que** la hauteur maximale (H₃) de l'hélice (28) peut valoir jusqu'à la dimension du diamètre extérieur de la tige de trocart (12).

5. Gaine de trocart selon l'une quelconque des revendications 2 à 4, **caractérisée en ce que** la hauteur (H) de l'hélice (28) diminue de nouveau jusque zéro, au plus tard à l'intérieur d'une spire de 360°, après avoir franchi la hauteur maximale (H₃).

6. Gaine de trocart selon l'une quelconque des revendications 1 à 5, **caractérisée en ce que** l'angle du pas de l'hélice (28), hors d'un plan perpendiculaire à l'axe de tige (13), en regardant de distal vers proximal, varie depuis un angle aigu supérieur à 45° jusqu'à un angle inférieur à 20°.

7. Gaine de trocart selon l'une quelconque des revendications 1 à 6, **caractérisée en ce qu'**il se trouve du côté distal de l'hélice (28) une partie de tige (24) sans filet extérieur.

8. Gaine de trocart selon l'une quelconque des revendications 1 à 7, **caractérisée en ce que** l'hélice (28) ne s'étend que dans une partie (25) de la tige (12).

9. Gaine de trocart selon l'une quelconque des revendications 1 à 8, **caractérisée en ce qu'**un contre-appui (34) déplaçable le long de l'axe de tige (13) est disposé sur la tige (12) du côté proximal de l'hélice (28), et peut être appliqué sur un côté supérieur d'un tissu, à travers lequel la gaine de trocart est enfoncée.

10. Gaine de trocart selon la revendication 9, **caractérisée en ce que** le contre-appui (34) est formé par un élément en forme de disque (36), qui est déplaçable le long de l'axe de tige (13) et qui peut être calé sur la tige (12) dans au moins une position.

11. Gaine de trocart selon une revendication 9 ou 10, **caractérisée en ce que** le contre-appui (34) présente un filet intérieur, qui peut être déplacé axialement en va-et-vient sur un côté extérieur (22) de la tige du côté proximal de l'hélice (28) au moyen d'un deuxième filet intérieur correspondant (30).

12. Gaine de trocart selon une revendication 9 ou 10, **caractérisée en ce que** le contre-appui (34) est fabriqué en un matériau à base de matière plastique élastique souple, et **en ce que** le contre-appui (34) peut être déplacé axialement en va-et-vient sur le côté extérieur (22) de la tige (12) au moyen d'un deuxième filet extérieur (30).

13. Gaine de trocart selon la revendication 12, **caractérisée en ce que** le contre-appui (34) est formé par un élément fendu, qui peut être pincé latéralement sur la tige (12).

14. Gaine de trocart selon l'une quelconque des revendications 11 à 13, **caractérisée en ce qu'**il se trouve une partie de tige sans filet (37) entre l'hélice (28) et le deuxième filet extérieur (30).

15. Gaine de trocart selon l'une quelconque des revendications 1 à 14, **caractérisée en ce que** la tige (12) présente un côté extérieur cylindrique (22).

16. Gaine de trocart selon l'une quelconque des revendications 1 à 15, **caractérisée en ce que** le premier filet extérieur (26) présente environ deux spires, dont la hauteur de pas totale correspond environ à l'épaisseur d'un tissu, à travers lequel la gaine de trocart doit être enfoncée en tournant.
